# EUROPÄISCHE PATENTSCHRIFT

(11) **EP 3 874 237 B1**
(45) Veröffentlichungstag und Bekanntmachung des Hinweises auf die Patenterteilung: **18.06.2025**
(21) Anmeldenummer: 19812663.3
(22) Anmeldetag: 30.10.2019
(51) Int. Cl.: G01F 1/22

(54) **VORRICHTUNG ZUR BESTIMMUNG VON PHYSIKALISCHEN MESSWERTEN, SCHWEBEKÖRPER UND EIN VERFAHREN**
DEVICE FOR DETERMINING PHYSICAL MEASUREMENT VALUES, FLOATING BODY AND A METHOD
DISPOSITIF DE DÉFINITION DE VALEURS DE MESURE PHYSIQUES, CORPS FLOTTEUR ET PROCÉDÉ

(30) Priorität: 01.11.2018 DE 102018008542
(43) Veröffentlichungstag der Anmeldung: 08.09.2021
(73) Patentinhaber: MFT MEISTER FLOW TECHNOLOGY HOLDING GMBH & CO. KG, 63831 Wiesen (DE)
(72) Erfinder: BECKER, Peter, 63831 Wiesen (DE); KEUPER, Melanie, 63831 Wiesen (DE)
(74) Vertreter: Nitz, Astrid
(86) Internationale Anmeldenummer: PCT/DE2019/000287
(87) Internationale Veröffentlichungsnummer: WO 2020/088706

(56) Entgegenhaltungen:
- EP-A1- 3 329 767
- CN-A- 107 677 516
- DE-A1- 102012 021 312
- GB-A- 2 005 421
- US-A- 4 935 726
- US-A1- 2014 157 886

## Beschreibung

Die Erfindung betrifft eine Vorrichtung zur Bestimmung von physikalischen Messwerten nach dem Oberbegriff von Anspruch 1, einen Schwebekörper nach Anspruch 10 und ein Verfahren zur Ausübung einer Messung nach dem Oberbegriff von Anspruch 11.

Bekannt ist es, eine Strömung mittels in Fluidströmungsleitungen für die Messung jeweils vorgesehenen angeordneten Schwebekörpern zu messen, wobei die Schwebekörperposition durch ein durchsichtiges Beobachtungsfenster im Messleitungsabschnitt. Nachteilig ist es, dass die Messeinrichtung unflexibel ist, sowohl in der Art und Ort der Anbringung als der Art der Messeinrichtung.

Aufgabe der vorliegenden Erfindung ist es, eine einfache und zuverlässige Vorrichtung zum Messen einer Materialströmung bereitzustellen, unter Vermeidung der Nachteile des Stands der Technik.

Die Aufgabe wird erfüllt durch eine Vorrichtung nach Anspruch 1.

Die vorliegende Erfindung ermöglicht es, auf jeweils spezifische, individuell angepasste Zusatzbaukomponenten und Sichtsysteme im Messleitungsabschnitt für Schwebekörper oder auch andere Bereiche einer Strömungsvorrichtung, für die Messwertaufnahme und Weitergabe verzichten zu können. Die Messwerte werden direkt im Schwebekörper aufgenommen und vorteilhaft auch gespeichert, so dass es zur sehr genauen und spezifischen Messungen im Medium genutzt werden kann. Zur Messwertaufnahme können vorteilhaft sehr viele unterschiedliche Sensormittel eingesetzt werden, wobei der Schwebekörper selbst als Sensor direkt im Medium messen kann, im Sinne eines intelligenten Signalmittels und zugleich vorteilhaft als Selbstversorger für die benötigte Energie im Sinne eines Energy-Harvesting arbeiten. Die gewonnenen Daten können vorteilhaft in übergeordnete Prozessleitsysteme übertragen werden, beispielsweise durch kontinuierliches sogenanntes Condition-Monitoring. Der Anwender kann auch in einem späteren Zeitpunkt nach dem Einbau Funktionseinheiten einfach entfernen oder hinzufügen. Die Vorrichtung ist sehr flexibel zu verwenden, für alle Arten von Materialströmungen, insbesondere auch für eine Suspension oder Emulsion von festen oder flüssigen Partikeln, oder auch in Form von strömendem Pulver. Durch im Voraus vorgesehene vielfältigen Mittel zum Anbringen und/oder Hindurchführen kann der Anwender die Sensorik modular einsetzen und/oder nachrüsten, mit unterschiedlichem Gewicht, Wanddicken, Material etc. Insbesondere können Materialien im Wesentlichen angepasst an die Eigenschaften der Materialströmung, insbesondere des Fluids, ausgewählt werden, insbesondere auch Materialgruppenübergreifend, so dass beispielsweise einerseits das Fluidum sicher geleitet wird und andererseits die Sensormittel im Schwebekörper stabil und sicher gehalten sind, wobei diese je nach Anforderungen des Verwenders einstückig gefertigt werden können. Durch die vorgeschlagene Vorrichtung können Sensoren mit ihren unterschiedlichsten Anforderung an die Art und Ausführung der Messwerterfassung im und/oder am Schwebekörper leicht integriert werden, auch bereits im Vorfeld einer Normenänderung, ohne dass der Schwebekörper ausgetauscht werden müsste. Der Schwebekörper kann einen Sensor umfassen, der beispielsweise direkt bei der Herstellung eingebracht ist und anschließend erst - bei Bedarf durch den Nutzer - durch eine abnehmbare Kontaktierung kontaktiert wird.

Vorteilhaft ist es, wenn ein Signalverarbeitungsmittel in und/oder am Schwebekörper vorgesehen ist.

Vorteilhaft ist es, wenn zumindest der Schwebekörper mittels eines additiven Fertigungsverfahrens, insbesondere 3D-Druckverfahrens, im Wesentlichen einstückig, aufzubauen ist und/oder zusammen mit dem Sensormittel integriert in den Schwebekörper und/oder Sensorsignalsendemittel und/oder Signalverarbeitungsmittel und/- oder Datenspeicher und/oder Energieversorgungsmittel, wobei insbesondere Schwebekörper als Inlay und/oder Hohlkörper umfassend Kunststoff auszubilden ist, wobei mittels 3D-Druck Sensormittel einzubetten sind und ein Inlay von einer Hülse umfassend ein Metall aufzunehmen und abzudichten ist.

Vorteilhaft ist es, wenn das Sensormittel integriert in den Schwebekörper und/oder Sensorsignalsendemittel und/oder Signalverarbeitungsmittel und/oder Datenspeicher und/oder Energieversorgungsmittel medienberührend und/oder beabstandet zum fließfähigen Medium, insbesondere ein Temperaturmesser mit einer Abstandsberechnung und/oder einem Temperaturgradienten des Schwebekörpermaterials, angeordnet sind.

Vorteilhaft ist es, wenn zur Kommunikation mit einem Empfangsmittel außerhalb des Schwebekörpers, insbesondere einem Datenauslesegerät, insbesondere im Messleitungsabschnitt angeordnet, für einen Empfang der Signale des Sensormittels.

Vorteilhaft ist es, wenn das ein Sensormittel zur Messung des Durchflusses und/oder des pH-Werts und/oder des Co₂-Gehalts und/oder der Fließgeschwindigkeit und/oder der momentanen Durchflussrate und/oder der Viskosität und/oder des Härtegrads und/oder des Mineralstoffgehalts, insbesondere mit einem RFID- und/oder NFC-Mittel und/oder einem Bluetooth-Sender, das insbesondere mit einer Dockingstation und/oder einem Computer und/oder Smartphone als Empfangsmittel gekoppelt ist.

Vorteilhaft ist es, wenn das Sensormittel, insbesondere mehrere Sensormittel, einen aktiven Sensor umfasst, durch den ein Spannungssignal zu erzeugen ist, wobei insbesondere mechanische und/oder chemische und/oder Licht- und/oder Wärmeenergie in ein Spannungssignal zu wandeln ist, insbesondere durch ein Thermoelement und/oder einen Lichtsensor und/oder einen Drucksensor und/oder das Sensormittel, insbesondere mehrere Sensormittel, einen passiven Sensor umfasst, wobei zusammen mit dem passiven Sensor ein Energieversorgungsmittel vorgesehen ist, um die Messgröße in ein elektrisch verwertbares Signal umzuwandeln, insbesondere durch einen induktiven und/oder kapazitiven und/oder optischen Sensor, insbesondere einen Reflexlichttaster und/oder eine Lichtschranke und/oder einen Magnetfeldsensor, insbesondere eine Hall-Sonde und/oder ein Reedkontakt, und/oder einen Ultraschallsensor.

Vorteilhaft ist es, wenn das fließfähige Medium eine Aerosolströmung und/oder Fluidströmung, insbesondere einer Gasströmung, insbesondere einer Luftströmung, und/oder einer Flüssigkeitsströmung umfasst.

Vorteilhaft ist es, wenn Energieversorgungsmittel einen Energiespeicher, insbesondere Akkumulator, und/oder Energiegewinnungsmittel umfasst, wobei eine Energieversorgung insbesondere über einen Hilfsstrom durch insbesondere Bewegungsenergie, insbesondere ein Flügelrad und/oder Rotation des Schwebekörpers, und/- oder über eine Verwertung einer Temperaturdifferenz, insbesondere ein Infrarotverfahren, und/oder Vibrationsenergie des Schwebekörpers, der vom fließfähigen Medium umströmbar und antreibbar ist und/oder über eine Induktionsspule im Schwebekörper.

Die Aufgabe wird ebenfalls gelöst durch ein Verfahren nach Anspruch 8.

Weitere Merkmale und Vorteile der Erfindung ergeben sich aus den Ansprüchen und der nachstehenden Beschreibung, in der Ausführungsbeispiele des Gegenstands der Erfindung in Verbindung mit den Zeichnungen näher erläutert sind.

Es zeigen:
- Fig. 1: einen schematischen Schnitt durch eine erfindungsgemäße Vorrichtung und
- Fig. 2: einen schematischen Schnitt durch eine erfindungsgemäße Vorrichtung.

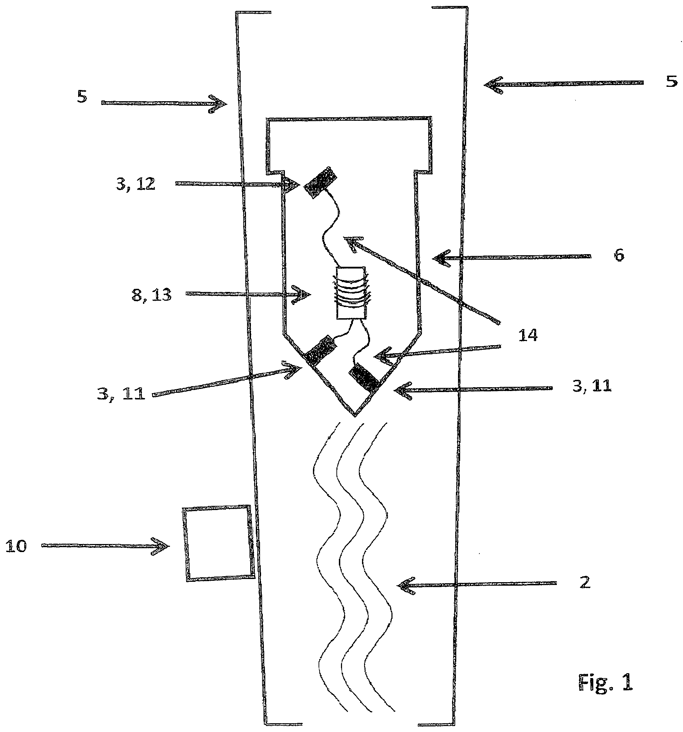

Fig. 1 zeigt einen schematischen Schnitt durch eine erfindungsgemäße Vorrichtung 1 zur Bestimmung von physikalischen Messwerten, die ein fließfähiges Medium 2 in einem Messleitungsabschnitt 5 und/oder eine Umgebung charakterisieren, wobei durch den Messleitungsabschnitt 5 das fließfähige Medium 2 hindurchströmen kann, und ein Schwebekörper 6 in dem Messleitungsabschnitt 5 im Wesentlichen frei angeordnet und vom fließfähigen Medium 2 umströmt und beweglich ist, insbesondere anhebbar, wobei in und/oder an dem Schwebekörper 6, insbesondere integriert in den Schwebekörper 6, zumindest ein, insbesondere mehrere, Sensormittel 3 angeordnet sind, zur Ausgabe eines Sensorsignals zur Feststellung eines physikalischen Messwerts, insbesondere Fließgeschwindigkeit und/oder Temperatur und/oder ph-Wert, und insbesondere im und/oder am Schwebekörper 6 ein Sensorsignalsendemittel 4 und/oder ein dem Sensormittel 3 zugeordneter Datenspeicher 7 vorgesehen ist, zur Weitergabe und/oder zumindest vorübergehenden Speicherung des Sensorsignals, und/oder insbesondere ein Energieversorgungsmittel 8 zur Versorgung des Sensormittels 3 mit Energie.

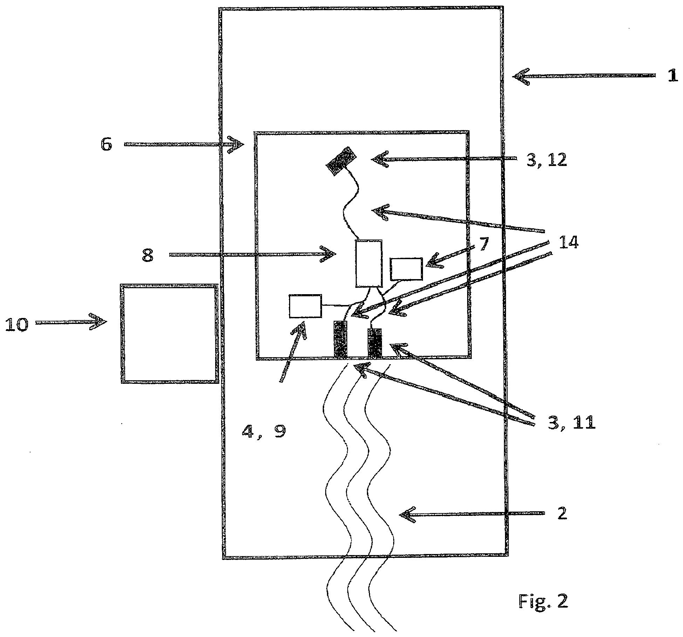

Fig. 2 zeigt einen schematischen Schnitt durch eine erfindungsgemäße Vorrichtung 1 wobei ein Signalverarbeitungsmittel 9 in und/oder am Schwebekörper 6 vorgesehen ist, wobei zumindest der Schwebekörper 6 mittels eines additiven Fertigungsverfahrens, insbesondere 3D-Druckverfahrens, im Wesentlichen einstückig, aufzubauen ist und/oder zusammen mit dem Sensormittel 3 integriert in den Schwebekörper 6 und/oder Sensorsignalsendemittel 4 und/oder Signalverarbeitungsmittel 9 und/oder Datenspeicher 7 und/oder Energieversorgungsmittel 8, wobei insbesondere Schwebekörper als Inlay und/oder Hohlkörper umfassend Kunststoff auszubilden ist, wobei mittels 3D-Druck Sensormittel 3 einzubetten sind und ein Inlay von einer Hülse umfassend ein Metall aufzunehmen und abzudichten ist, wobei das Sensormittel 3 integriert in den Schwebekörper 6 und/oder Sensorsignalsendemittel 4 und/oder Signalverarbeitungsmittel 9 und/oder Datenspeicher 7 und/oder Energieversorgungsmittel 8 medienberührend und/oder beabstandet zum fließfähigen Medium 2, insbesondere ein Temperaturmesser mit einer Abstandsberechnung und/oder einem Temperaturgradienten des Schwebekörpermaterials, angeordnet sind, wobei zur Kommunikation mit einem Empfangsmittel 10 außerhalb des Schwebekörpers 6, insbesondere einem Datenauslesegerät, insbesondere im Messleitungsabschnitt 5 angeordnet, für einen Empfang der Signale des Sensormittels 3. Das Sensormittel 3 kann eingesetzt werden zur Messung des Durchflusses und/oder des pH-Werts und/oder des Co₂-Gehalts und/oder der Fließgeschwindigkeit und/oder der momentanen Durchflussrate und/oder der Viskosität und/oder des Härtegrads und/oder des Mineralstoffgehalts, insbesondere mit einem RFID- und/oder NFC-Mittel und/oder einem Bluetooth-Sender, das insbesondere mit einer Dockingstation und/oder einem Computer und/oder Smartphone als Empfangsmittel 10 gekoppelt ist. Ein Sensormittel 3 ist insbesondere medienberührend 11 oder nicht medienberührend 12 und mittels einer Stromleitung 14 an ein Energieversorgungsmittel 8, insbesondere eine Induktionsspule 13 oder ein Signalverarbeitungsmittel 9 oder einen Datenspeicher 7 gekoppelt.

Das Sensormittel 3, insbesondere mehrere Sensormittel, kann beispielsweise einen aktiven Sensor umfassen, durch den ein Spannungssignal zu erzeugen ist, wobei insbesondere mechanische und/oder chemische und/oder Licht- und/oder Wärmeenergie in ein Spannungssignal zu wandeln ist, insbesondere durch ein Thermoelement und/oder einen Lichtsensor und/oder einen Drucksensor und/oder das Sensormittel 3, insbesondere mehrere Sensormittel, einen passiven Sensor, wobei zusammen mit dem passiven Sensor ein Energieversorgungsmittel 8 vorgesehen ist, um die Messgröße in ein elektrisch verwertbares Signal umzuwandeln, insbesondere durch einen induktiven und/oder kapazitiven und/oder optischen Sensor, insbesondere einen Reflexlichttaster und/oder eine Lichtschranke und/oder einen Magnetfeldsensor, insbesondere eine Hall-Sonde und/oder ein Reedkontakt, und/oder einen Ultraschallsensor.

Das fließfähige Medium 2 kann eine Aerosolströmung und/oder Fluidströmung umfassen, insbesondere einer Gasströmung, insbesondere einer Luftströmung, und/oder einer Flüssigkeitsströmung.

### BEZUGSZEICHENLISTE

- 1: Vorrichtung zur Bestimmung von physikalischen Messwerten
- 2: fließfähiges Medium
- 3: Sensormittel
- 4: Sensorsignalsendemittel
- 5: Messleitungsabschnitt
- 6: Schwebekörper
- 7: Datenspeicher
- 8: Energieversorgungsmittel
- 9: Signalverarbeitungsmittel
- 10: Empfangsmittel
- 11: medienberührend
- 12: nicht medienberührend
- 13: Induktionsspule
- 14: Stromleitung

## Patentansprüche

1. Vorrichtung (1) zur Bestimmung von physikalischen Messwerten, die ein fließfähiges Medium (2) in einem Messleitungsabschnitt (5) und/oder eine Umgebung charakterisieren, wobei durch den von der Vorrichtung umfassten Messleitungsabschnitt (5) das fließfähige Medium (2) hindurchströmen kann, und ein Schwebekörper (6) in dem Messleitungsabschnitt (5) im Wesentlichen frei angeordnet und vom fließfähigen Medium (2) umströmt und beweglich ist, nämlich anhebbar, wobei in und/oder an dem Schwebekörper (6), nämlich integriert in den Schwebekörper (6), zumindest ein, insbesondere mehrere, Sensormittel (3) angeordnet sind, zur Ausgabe eines Sensorsignals zur Feststellung eines physikalischen Messwerts, insbesondere Fließgeschwindigkeit und/oder Temperatur und/oder ph-Wert, und insbesondere im Schwebekörper (6) ein Sensorsignalsendemittel (4) und/oder ein dem Sensormittel (3) zugeordneter Datenspeicher (7) vorgesehen ist, zur Weitergabe und/oder zumindest vorübergehenden Speicherung des Sensorsignals, und/oder insbesondere ein Energieversorgungsmittel (8) zur Versorgung des Sensormittels (3) mit Energie, wobei ein Signalverarbeitungsmittel (9) in und/oder am Schwebekörper (6) vorgesehen ist, wobei zumindest der Schwebekörper (6) mittels eines additiven Fertigungsverfahrens, nämlich 3D-Druckverfahrens, im Wesentlichen einstückig, aufzubauen ist und/oder zusammen mit dem Sensormittel (3) integriert in den Schwebekörper (6) und Sensorsignalsendemittel (4) und Signalverarbeitungsmittel (9) und Datenspeicher (7) und Energieversorgungsmittel (8), wobei der Schwebekörper als Inlay und/oder Hohlkörper umfassend Kunststoff auszubilden ist, wobei mittels 3D-Druck Sensormittel (3) einzubetten sind und ein Inlay von einer Hülse umfassend ein Metall aufzunehmen und abzudichten ist, wobei der Schwebekörper als Selbstversorger im Sinne eines Energy-Harvesting vorgesehen ist.

2. Vorrichtung nach Anspruch 1, **dadurch gekennzeichnet, dass** das Sensormittel (3) integriert in den Schwebekörper (6) und/oder Sensorsignalsendemittel (4) und/oder Signalverarbeitungsmittel (9) und/oder Datenspeicher (7) und/oder Energieversorgungsmittel (8) medienberührend und/oder beabstandet zum fließfähigen Medium (2), insbesondere ein Temperaturmesser mit einer Abstandsberechnung und/oder einem Temperaturgradienten des Schwebekörpermaterials, angeordnet sind.

3. Vorrichtung nach einem der Ansprüche 1 oder 2 **dadurch gekennzeichnet, dass** zur Kommunikation mit einem Empfangsmittel (10) außerhalb des Schwebekörpers (6), insbesondere einem Datenauslesegerät, insbesondere im Messleitungsabschnitt (5) angeordnet, für einen Empfang der Signale des Sensormittels (3).

4. Vorrichtung nach einem der Ansprüche 1 bis 3, **dadurch gekennzeichnet, dass** das ein Sensormittel (3) zur Messung des Durchflusses und/oder des pH-Werts und/oder des Co₂-Gehalts und/oder der Fließgeschwindigkeit und/oder der momentanen Durchflussrate und/oder der Viskosität und/oder des Härtegrads und/oder des Mineralstoffgehalts, insbesondere mit einem RFID- und/oder NFC-Mittel und/oder einem Bluetooth-Sender, das insbesondere mit einer Dockingstation und/oder einem Computer und/oder Smartphone als Empfangsmittel (10) gekoppelt ist.

5. Vorrichtung nach einem der Ansprüche 1 bis 4, **dadurch gekennzeichnet, dass** das Sensormittel (3), insbesondere mehrere Sensormittel, einen aktiven Sensor umfasst, durch den ein Spannungssignal zu erzeugen ist, wobei insbesondere mechanische und/oder chemische und/oder Licht- und/oder Wärmeenergie in ein Spannungssignal zu wandeln ist, insbesondere durch ein Thermoelement und/oder einen Lichtsensor und/oder einen Drucksensor und/oder das Sensormittel (3), insbesondere mehrere Sensormittel, einen passiven Sensor umfasst, wobei zusammen mit dem passiven Sensor ein Energieversorgungsmittel (8) vorgesehen ist, um die Messgröße in ein elektrisch verwertbares Signal umzuwandeln, insbesondere durch einen induktiven und/oder kapazitiven und/oder optischen Sensor, insbesondere einen Reflexlichttaster und/oder eine Lichtschranke und/oder einen Magnetfeldsensor, insbesondere eine Hall-Sonde und/oder ein Reedkontakt, und/oder einen Ultraschallsensor.

6. Vorrichtung nach einem der Ansprüche 1 bis 5, **dadurch gekennzeichnet, dass** das fließfähige Medium (2) eine Aerosolströmung und/oder Fluidströmung, insbesondere einer Gasströmung, insbesondere einer Luftströmung, und/oder einer Flüssigkeitsströmung umfasst.

7. Vorrichtung nach einem der Ansprüche 1 bis 6, **dadurch gekennzeichnet, dass** Energieversorgungsmittel (8) einen Energiespeicher, insbesondere Akkumulator, und/oder Energiegewinnungsmittel umfasst, wobei eine Energieversorgung insbesondere über einen Hilfsstrom durch insbesondere Bewegungsenergie, insbesondere ein Flügelrad und/oder Rotation des Schwebekörpers (6), und/oder über eine Verwertung einer Temperaturdifferenz, insbesondere ein Infrarotverfahren, und/oder Vibrationsenergie des Schwebekörpers (6), der vom fließfähigen Medium (2) umströmbar und antreibbar ist und/oder über eine Induktionsspule im Schwebekörper (6).

8. Verfahren zur Ausübung einer Messung von physikalischen Eigenschaften eines fließfähigen Mediums (2) und seiner Umgebung, wobei eine Vorrichtung (1) nach einem der vorstehenden Ansprüche zur Bestimmung von physikalischen Messwerten, die ein fließfähiges Medium (2) in einem Messleitungsabschnitt (5) und/oder eine Umgebung charakterisieren, verwendet wird, wobei durch den Messleitungsabschnitt (5) das fließfähige Medium (2) hindurchströmt, und ein Schwebekörper (6) in dem Messleitungsabschnitt (5) im Wesentlichen frei angeordnet und vom fließfähigen Medium (2) umströmt und beweglich ist, insbesondere angehoben wird, wobei in und/oder an dem Schwebekörper (6), insbesondere integriert in den Schwebekörper (6), zumindest ein, insbesondere mehrere, Sensormittel (3) angeordnet werden, zur Ausgabe eines Sensorsignals zur Feststellung eines physikalischen Messwerts, insbesondere Fließgeschwindigkeit und/oder Temperatur und/oder ph-Wert, und insbesondere im und/oder am Schwebekörper (6) ein Sensorsignalsendemittel (4) und/oder ein dem Sensormittel (3) zugeordneter Datenspeicher (7) vorgesehen ist, zur Weitergabe und/oder zumindest vorübergehenden Speicherung des Sensorsignals, und/oder insbesondere ein Energieversorgungsmittel (8) zur Versorgung des Sensormittels (3) mit Energie, wobei der Schwebekörper als Selbstversorger für die benötigte Energie im Sinne eines Energy-Harvesting betrieben wird.

## Claims

1. Device (1) for determining physical measurement values that characterize a flowable medium (2) in a measuring pipe section (5) and/or an environment, wherein the flowable medium (2) can flow through the measuring pipe section (5) encompassed by the device, and a floating body (6) is arranged substantially freely in the measuring pipe section (5) and is surrounded by the flowable medium (2) and is movable, namely lift-able, wherein at and/or in the floating body (6), namely integrated in the floating body (6), at least one, in particular several, sensor means (3) are arranged, for outputting a sensor signal for determining a physical measurement value, in particular flow speed and/or temperature and/or pH value, and in particular in the floating body (6) a sensor signal transmission means (4) and/or assigned to the sensor means (3) a data memory (7) is provided for the transmission and/or at least temporary storage of the sensor signal, and/or in particular a power supply means (8) for supplying the sensor means (3) with energy, wherein a signal processing means (9) is provided in and/or on the floating body (6), where at least the floating body (6) is to be built essentially in one piece by an additive manufacturing process, namely a 3D printing process, and/or integrated with the sensor means (3) in the floating body (6) and the sensor signal transmission means (4) and signal processing means (9) and data memory (7) and power supply means (8), where the floating body is to be designed as an inlay and/or hollow body comprising plastic, where sensor means (3) are to be embedded by 3D printing and an inlay of a sleeve comprising a metal to be picked up and sealed, with the floating body being a self-sufficient energy harvester.

2. Device according to claim 1, **characterized in that** the sensor means (3) is integrated into the floating body (6) and/or the sensor signal transmitting means (4) and/or the signal processing means (9) and/or the data storage means (7) and/or the power supply means (8) are in contact with and/or spaced from the flowable medium (2), in particular a temperature measuring device with a distance calculation and/or a temperature gradient of the floating body material.

3. Device according to one of claims 1 or 2, **characterized in that** for communication with a receiving means (10) outside the floating body (6), in particular a data reading device, in particular arranged in the measuring pipe section (5), for receiving the signals of the sensor means (3).

4. Device according to one of claims 1 to 3, **characterized in that** it comprises a sensor means (3) for measuring the flow and/or the pH value and/or the CO2 content and/or the flow velocity and/or the instantaneous flow rate and/or the viscosity and/or the hardness and/or the mineral content, in particular with an RFID and/or NFC means and/or a Bluetooth transmitter, which is in particular coupled with a docking station and/or a computer and/or smartphone as receiving means (10).

5. Device according to one of claims 1 to 4, **characterized in that** the sensor means (3), in particular several sensor means, comprises an active sensor, by which a voltage signal is to be generated, in particular mechanical and/or chemical and/or light and/or heat energy is to be converted into a voltage signal, in particular by a thermocouple and/or a light sensor and/or a pressure sensor, and/or the sensor means (3), in particular several sensor means, comprises a passive sensor, by which, together with the passive sensor, an energy supply means (8) is provided to convert the measurable quantity into an electrically usable signal, in particular by an inductive and/or capacitive and/or optical sensor, in particular a reflex light sensor and/or a light barrier and/or a magnetic field sensor, in particular a Hall probe and/or a reed contact, and/or an ultrasonic sensor.

6. Device according to one of claims 1 to 5, **characterized in that** the flowable medium (2) comprises an aerosol flow and/or fluid flow, in particular a gas flow, in particular an air flow, and/or a liquid flow.

7. Device according to one of claims 1 to 6, **characterized in that** the energy supply means (8) comprise an energy storage, in particular an accumulator, and/or energy generation means, wherein the energy supply is provided in particular by an auxiliary current through in particular kinetic energy, in particular a flywheel and/or rotation of the floating body (6), and/or by utilizing a temperature difference, in particular an infrared method, and/or vibration energy of the floating body (6), which can be flowed around and driven by the fluid medium (2) and/or by an induction coil in the floating body (6).

8. Method for measuring physical properties of a fluid medium (2) and its surroundings, wherein a device (1) according to one of the preceding claims is used to determine physical measurement values characterizing a fluid medium (2) in a measuring pipe section (5) and/or surroundings, wherein the fluid medium (2) flows through the measuring pipe section (5), and a floating body (6) in the measuring pipe section (5) is substantially freely arranged and surrounded by the fluid medium (2) and is movable, in particular is raised wherein at and/or in the floating body (6), particularly integrated in the floating body (6), at least one, particularly several, sensor means (3) are arranged, for outputting a sensor signal for determining a physical measured value, particularly flow velocity and/or temperature and/or pH value, and particularly in and/or at the floating body (6) a sensor signal transmission means (4) and/or a data memory (7) assigned to the sensor means (3) is provided, for forwarding and/or at least temporary storage of the sensor signal, and/or particularly an energy supply means (8) for supplying the sensor means (3) with energy, wherein the floating body is operated as a self-supplier for the required energy in the sense of an energy harvesting.

## Revendications

1. Dispositif (1) permettant de déterminer des valeurs de mesure physiques caractérisant un fluide (2) dans une section de conduite de mesure (5) et/ou un environnement, le fluide (2) pouvant circuler à travers la section de conduite de mesure (5) comprise par le dispositif, et un corps flottant (6) dans la section de conduite de mesure (5) étant essentiellement librement disposé et entouré par le fluide (2) et pouvant être déplacé, à savoir soulevable, au moins un, en particulier plusieurs, moyens de détection (3) étant disposés dans et/ou sur le corps flottant (6), en particulier intégrés dans le corps flottant (6), pour émettre un signal de capteur permettant de déterminer une valeur de mesure physique, en particulier la vitesse d'écoulement et/ou la température et/ou le pH, et en particulier dans le corps flottant (6) un moyen d'émission de signal de capteur (4) et/ou une mémoire de données (7) associée au moyen de détection (3) étant prévue.pour transmettre et/ou stocker temporairement le signal du capteur, et/ou en particulier un moyen d'alimentation (8) pour alimenter le moyen de capteur (3) en énergie, un moyen de traitement du signal (9) étant prévu dans et/ou sur le corps flottant (6), où au moins le corps flottant (6) doit être construit essentiellement en une seule pièce au moyen d'un procédé de fabrication additive, à savoir un procédé d'impression 3D, et/ou intégré avec le moyen capteur (3) dans le corps flottant (6) et le moyen d'émission de signaux capteurs (4) et le moyen de traitement de signaux (9) et le moyen de stockage de données (7) et le moyen d'alimentation en énergie (8), le corps flottant devant être conçu en plastique en tant qu'inlay et/ou corps creux, les moyens capteurs (3) devant être intégrés au moyen d'impression 3D et un inlay d'une douille comprenant un métal.

2. Dispositif selon la revendication 1, **caractérisé en ce que** le moyen de détection (3) est intégré au corps flottant (6) et/ou au moyen d'émission de signaux de détection (4) et/ou au moyen de traitement de signaux (9) et/ou au moyen de stockage de données (7) et/ou au moyen d'alimentation en énergie (8) en contact avec le milieu fluide (2) et/ou à distance de celui-ci, en particulier un thermomètre avec un calcul de distance et/ou un gradient de température du matériau du corps flottant.

3. Dispositif selon l'une des revendications 1 ou 2, **caractérisé en ce que** pour la communication avec un moyen de réception (10) situé à l'extérieur du corps flottant (6), en particulier un dispositif de lecture de données, en particulier dans la section de la ligne de mesure (5), pour recevoir les signaux du moyen de détection (3).

4. Dispositif selon l'une des revendications 1 à 3, **caractérisé en ce que** le moyen de détection (3) est destiné à mesurer le débit et/ou le pH et/ou la teneur en CO2 et/ou la vitesse d'écoulement et/ou le débit instantané et/ou la viscosité et/ou le degré de dureté et/ou la teneur en minéraux, en particulier avec un moyen RFID et/ou NFC et/ou un émetteur Bluetooth, qui est en particulier couplé à une station d'accueil et/ou un ordinateur et/ou un smartphone en tant que moyen de réception (10).

5. Dispositif selon l'une des revendications 1 à 4, **caractérisé en ce que** le moyen de détection (3), en particulier plusieurs moyens de détection, comprend un capteur actif, par lequel un signal de tension est généré, en particulier une énergie mécanique et/ou chimique et/ou lumineuse et/ou thermique est transformée en un signal de tension, en particulier par un thermocouple et/ou un capteur de lumière et/ou un capteur de pression et/ou le moyen de détection (3), en particulier plusieurs moyens de détection, comprend un capteur passif, par lequel, avec le capteur passif, un moyen d'alimentation en énergie (8) est prévu pour transformer la grandeur de mesure en un signal utilisable électriquement, en particulier par un capteur inductif et/ou capacitif et/ou optique, en particulier un capteur de réflexion lumineuse et/ou une barrière lumineuse et/ou un capteur de champ magnétique, en particulier une sonde Hall et/ou un Reedkontakt et/ou un capteur ultrasonore.

6. Dispositif selon l'une quelconque des revendications 1 à 5, **caractérisé en ce que** le fluide (2) comprend un flux d'aérosol et/ou un flux de fluide, en particulier un flux de gaz, en particulier un flux d'air, et/ou un flux de liquide.

7. Dispositif selon l'une des revendications 1 à 6, **caractérisé en ce que** les moyens d'alimentation en énergie (8) comprennent un accumulateur et/ou des moyens de production d'énergie, notamment un générateur d'énergie, en particulier par le biais d'un courant auxiliaire, par exemple par l'énergie cinétique, notamment un volant d'inertie et/ou la rotation du corps flottant (6), et/ou par l'exploitation d'une différence de température, notamment un procédé infrarouge, et/ou l'énergie vibratoire du corps flottant (6), qui peut être mis en rotation et entraîné par le fluide (2) et/ou par une bobine d'induction dans le corps flottant (6).

8. Procédé pour effectuer une mesure des propriétés physiques d'un fluide (2) et de son environnement, une unité (1) selon l'une des revendications précédentes étant utilisée pour déterminer les valeurs de mesure physiques caractérisant un fluide (2) dans une section de conduite de mesure (5) et/ou un environnement, le fluide (2) traversant la section de conduite de mesure (5), et un corps flottant (6) dans la section de conduite de mesure (5) étant essentiellement librement disposé et entouré par le fluide (2) et pouvant se déplacer, en particulier s'élever, dans ce cas, au moins un, en particulier plusieurs, moyens de détection (3) sont disposés dans et/ou sur le corps flottant (6), en particulier intégrés dans le corps flottant (6), pour émettre un signal de détection permettant de déterminer une valeur de mesure physique, en particulier la vitesse d'écoulement et/ou la température et/ou le pH, et en particulier dans et/ou sur le corps flottant (6) un moyen d'émission de signal de détection (4) et/ou une mémoire de données (7) associée au moyen de détection (3) sont prévus, pour transmettre et/ou stocker temporairement le signal de détection, et/ou en particulier un moyen d'alimentation (8) pour alimenter le moyen de détection (3) en énergie, le corps flottant étant exploité comme un auto-alimentateur pour l'énergie nécessaire au sens d'un Energy-Harvesting.
